Europäisches Patentamt

**European Patent Office**

**Office européen des brevets**

(11) Veröffentlichungsnummer : **0 100 340**
**B1**

(12) **EUROPÄISCHE PATENTSCHRIFT**

(45) Veröffentlichungstag der Patentschrift :
21.11.85

(21) Anmeldenummer : 83900612.9

(22) Anmeldetag : 11.02.83

(86) Internationale Anmeldenummer :
PCT/AT 83/00005

(87) Internationale Veröffentlichungsnummer :
WO/8302717 (18.08.83 Gazette 83/19)

(51) Int. Cl.⁴ : **A 61 B 3/12**

(54) **OPHTALMOSKOPISCHES GERÄT.**

(30) Priorität : 12.02.82 DE 3205107

(43) Veröffentlichungstag der Anmeldung :
15.02.84 Patentblatt 84/07

(45) Bekanntmachung des Hinweises auf die Patenterteilung : 21.11.85 Patentblatt 85/47

(84) Benannte Vertragsstaaten :
AT CH DE FR GB LI NL SE

(56) Entgegenhaltungen :
DE-A- 2 301 468
DE-C-   485 096
GB-A-   784 371

(73) Patentinhaber : STEINHUBER, Wolfdietrich
Hofwaldweg 10
A-6020 Innsbruck (AT)

(72) Erfinder : STEINHUBER, Wolfdietrich
Hofwaldweg 10
A-6020 Innsbruck (AT)

(74) Vertreter : Hofinger, Engelbert et al
Torggler-Hofinger Wilhelm-Greil-Strasse 16
A-6020 Innsbruck (AT)

## Beschreibung

Die Erfindung betrifft ein ophthalmoskopisches Gerät zur abschnittsweisen Untersuchung und Beobachtung des Augenhintergrundes, insbesondere des nichtzentralen Bereiches, mit einem ein Objektiv aufweisenden und ein Beobachtungstrahlenbündel erzeugenden optischen System, mit einer Beleuchtungseinrichtung, deren Strahlenbündel das Objektiv im wesentlichen koaxial zum Beobachtungsstrahlenbündel verläßt, und mit einem exzentrisch zur Achse des Gerätes angeordneten Fixierobjekt zur Ausrichtung der Augenachse, wobei durch die Geräteachse und das Fixierobjekt eine Ebene definiert ist.

Für die Untersuchung des Augenhintergrundes werden direkte oder indirekte Augenspiegel verwendet, die sowohl in einfacher als auch in aufwendiger Ausführung mit Zusatzeinrichtungen (Kamera usw.) vor allem dazu geeignet sind, den Augenhintergrund im Winkel von etwa 60° um die optische Achse zu betrachten. Augenspiegel, die von Hand frei zu halten sind, können nach entsprechender Übung mit viel Geschick auch so schräg an die Linse des zu untersuchenden Auges angenähert werden, daß ein vergrößerter Bereich der Netzhaut betrachtet werden kann, doch ist keine exakte Untersuchung möglich.

Es ist weiters bekannt, an ophthalmologischen Geräten ein stark sammelndes Immersionsobjektiv vorzusehen, das unmittelbar auf die Hornhaut aufgelegt wird. Hierdurch kann ein wesentlich größerer Bereich des Augenhintergrundes betrachtet werden, doch stellt die Auflage eines Objektivs auf die Hornhaut für die meisten Patienten eine im höchsten Maße unangenehme Untersuchungsmethode dar. In streuenden Immersionsobjektiven können auch Spiegel angeordnet sein, sodaß ein größerer Netzhautbereich erfaßbar ist.

Die Erfindung hat es sich nun zur Aufgabe gestellt, ein ophthalmoskopisches Gerät zu schaffen, mittels dessen auch der periphere Bereich des Augenhintergrundes betrachtet werden kann, ohne daß ein Objektiv auf die Hornhaut aufgelegt werden muß. Weiters soll der periphere Bereich des Augenhintergrundes auch weitgehend verzerrungsfrei abgebildet bzw. wiedergegeben werden können.

Zur Lösung dieser Aufgabe ist nun erfindungsgemäß bei einem ophthalmoskopischen Gerät der eingangs genannten Art vorgesehen, daß das Gerät als Standgerät mit einem verstellbaren Objektiv ausgebildet ist, wobei dem Objektiv zumindest eine verstellbare Einrichtung zur Umlenkung der beiden Strahlenbündel zugeordnet ist, mittels der das Bild der Lichtquelle und das Bild der Pupille des Untersuchers unter unterschiedlichen Betrachtungswinkeln in die Pupille des zu untersuchenden Auges legbar ist, wenn der Mittelpunkt des auf das Fixierobjekt gerichteten Auges in der Geräteachse liegt, und daß das optische System, die Beleuchtungseinrichtung, das Fixierobjekt und die Umlenkeinrichtung gemeinsam um die Geräteachse drehbar sind.

Die Umlenkeinrichtung kann dabei beispielsweise durch zumindest zwei verstellbare Umlenkflächen gebildet sein, die mittels einer Verstelleinrichtung aus einer Grundstellung in mehrere Stellungen bewegbar sind, in denen jeweils ein exzentrischer Ausschnitt des Augenhintergrundes betracht- bzw. untersuchbar ist. Durch Drehen des Gerätes um seine Achse ergänzen sich die betrachteten Ausschnitte zu Ringen, da das sich mitdrehende Fixierobjekt insbesondere ein Fixierlicht, nach allen Richtungen dieselbe Schrägstellung der Augenachse bewirkt. Nach der Betrachtung aller Ausschnitte eines Ringes können durch Veränderung der Umlenkflächen in die nächste Stellung die Ausschnitte eines anschließenden Ringes betrachtet werden, sodaß mit abwechselnder Verdrehung des Gerätes und Verstellung der Umlenkflächen der Augenhintergrund im wesentlichen vollständig erfaßt werden kann. Bevorzugte Stellungen der Umlenkflächen bewirken beispielsweise Betrachtungswinkel von 0°-17°, 17°-45°, 45°-67° und von mehr als 67° von der Augenachse, wobei in der ersten Stellung, die auch die Justierung aller bewegbaren Teile ermöglicht, die Augenachse mit der Geräteachse zusammenfällt, während in allen anderen Stellungen das Auge auf das Fixierobjekt gerichtet ist, das etwa in einem Winkel von 40° von der Geräteachse dem zu untersuchenden Auge erscheint.

Die Umlenkflächen, die insbesondere durch Umlenkspiegel gebildet sind, können einzeln oder gemeinsam verschiebbar oder kippbar sein. Beispielsweise ist in einer Ausführung vorgesehen, daß von zwei Umlenkflächen die dem Objektiv nähere Umlenkfläche um eine Achse kippbar und die dem Auge nähere Umlenkfläche auf zwei nichtparallelen Führungsbahnen schwenkbar ist.

Da einerseits durch die einfache Verschiebung der augennahen Umlenkfläche eine Wegverlängerung des Strahlenbündels erfolgt, und andererseits der vordere Abbildungspunkt der Lichtquelle ortsunverändert bleiben soll, ist es zur Vereinfachung der Schiebebahn bzw. Schiebebewegung günstig, wenn auch das Objektiv in seiner Lage verändert, beispielsweise längs der Geräteachse verschoben wird. Es kann zu diesem Zweck auch ein Wechselobjektiv vorgesehen sein, das zumindest zwei unterschiedliche Linsen in einem drehbaren Käfig aufweist, dessen Drehachse senkrecht zur Geräteachse und zu der durch diese und das Fixierobjekt definierten Ebene verläuft. Mit zunehmendem Betrachtungswinkel steht der zu betrachtende Ausschnitt des Augenhintergrundes schräg zu den Strahlenbündeln, sodaß keine tiefenscharfe Abbildung erzielt werden kann. Um nun dennoch eine zumindest annähernd tiefenscharfe Abbildung zu erzielen, ist in einer weiteren Ausführung der Erfindung dem Objektiv ein kippbares Zwischenbildlinsensystem zugeordnet, mittels der ein entzerrtes Zwischenbild erzielt werden kann. Das Zwischenlinsensystem, das Objektiv und die Umlenkeinrichtung können dabei in ihrer Bewegung mechanisch gekoppelt sein. Da das Zwischenlinsensystem entzerrend wirkt, kann dieser Effekt zusätzlich dazu genützt werden, eine Verzerrung durch das Objektiv aufzuheben, sodaß dieses geringfügig geneigt und gegebenenfalls auch kippbar angeordnet werden kann. Auch diese Bewegung kann in den Bewegungsablauf der anderen Bauteile mechanisch eingekoppelt sein, sodaß bei der Verstellung der Umlenkeinrichtung beim Übergang auf den nächsten Betrachtungsring keine zusätzliche Justierung der übrigen verstellbaren Teile erforderlich ist.

Da im Beleuchtungssystem das Bild der Lichtquelle aufgrund des gekippten Objektivs verzerrt ist und dadurch störende Reflexe erzeugt, ist in einer weiteren bevorzugten Ausführung vorgesehen, daß auch dem Beleuchtungsstrahlenbündel eine entzerrtes Zwischenbildlinsensystem zugeordnet ist, das gegebenenfalls ebenfalls verstellbar ist.

Weiters können durch das Einspiegeln eines Vergleichsbildes, beispielsweise eines Fundusfotos einer vorhergehenden Untersuchung des Patienten, Veränderungen der Netzhaut leicht kontrolliert werden. Auch könnten mehrere Fotos ineinander projiziert werden, wobei diese Fotos in das Beobachtungs- und/oder das Beleuchtungsstrahlenbündel einspiegelbar sind. Das erfindungsgemäße Gerät kann auch mit bekannten Zusatzeinrichtungen wie einer Kamera, einem Stereoaufsatz, einer Laser- oder Lichtkoagulationseinrichtung usw. kombiniert werden.

Nachstehend wird nun die Erfindung anhand der Figuren der beiliegenden Zeichnungen näher beschrieben, ohne darauf beschränkt zu sein.

Es zeigen :

Figur 1 einen Längsschnitt durch eine bevorzugte Ausführung eines erfindungsgemäßen Gerätes in einer Ausgangsstellung ohne Verwendung der Umlenkeinrichtung,

Figur 2 eine vergrößerte schematische Darstellung des Beobachtungs- bzw. Beleuchtungsstrahlenganges in einer anderen Ausgangsstellung unter Verwendung der Umlenkeinrichtung,

Figuren 3 bis 6 schematische Einzeldarstellungen verschiedener Stellungen der Umlenkspiegel,

Figur 7 ein Schaubild der verschiedenen Stellungen der Umlenkspiegel, und

Figur 8 einen Längsschnitt durch eine weitere Ausführung mit einer abgeänderten Umlenkeinrichtung.

Das Gerät gemäß einem ersten Ausführungsbeispiel der Erfindung weist ein im wesentlichen zylindrisches Gehäuse 1 auf, das in einem Lager 29 eines Gestells 17 um die Geräteachse 8 drehbar gelagert ist. Das Gestell 17 steht verstellbar auf einem Tisch 16 und ist mit einer Kinnauflage 20 versehen, sodaß das Gerät gegenüber dem zu untersuchenden Auge 7 so eingerichtet werden kann, daß die geometrische Geräteachse 8 durch den Mittelpunkt A des Auges 7 verläuft.

Im Gehäuse 1 ist ein aus einer Linsenkombination bestehendes Objektiv 2 mittels einer Verschiebeeinrichtung 22 in der Geräteachse 8 verschiebbar und um eine Achse 12 kippbar angeordnet. Außerhalb des Gehäuses 1 ist eine Beleuchtungseinrichtung 5 vorgesehen, deren Licht im Gehäuse 1 umgelenkt und dort über eine Umlenkfläche 23 auf das Objektiv 2 geworfen wird. Das Beleuchtungsstrahlenbündel verläuft in der Ausgangsstellung vom Objektiv 2 koaxial zur Geräteachse 8, sodaß das Bild B der Lichtquelle bei richtiger Justierung des Gerätes in den Mittelpunkt der Augenlinse 21 verlegt ist. Die durch die Augenlinse eintretenden Strahlen beleuchten je nach Einfallswinkel einen bestimmten Bereich des Augenhintergrundes. Der Strahlengang des Beobachtungsstrahlenbündels erstreckt sich vom Okular 30, das bevorzugt mit einem Stereoaufsatz 19 bestückt ist, der zur Bildaufrichtung bevorzugt ein die Drehung des optischen Systems um die Geräteachse 8 ausgleichendes, drehendes Dove-Prisma enthält, durch ein Zwischenbildlinsensystem 6 zum Objektiv 2, wobei das Beobachtungsstrahlenbündel im wesentlichen koaxial zum Beleuchtungsstrahlenbündel verläuft. Im wesentlichen koaxial schließt hier einen sehr kleinen Winkel zwischen den beiden Strahlenbündeln ein, unter dem sie, um Reflexreduktion zu erzielen, auf das Objektiv 2 auftreffen. Der vordere Strahlengang des Beobachtungsbündels entspricht daher im wesentlichen dem des Beleuchtungsbündels. Über eine einklappbare Umlenkfläche 24 kann das Beobachtungsstrahlenbündel auf einen Fotoaufsatz 18 mit lichtempfinglicher Schicht umgeleitet werden.

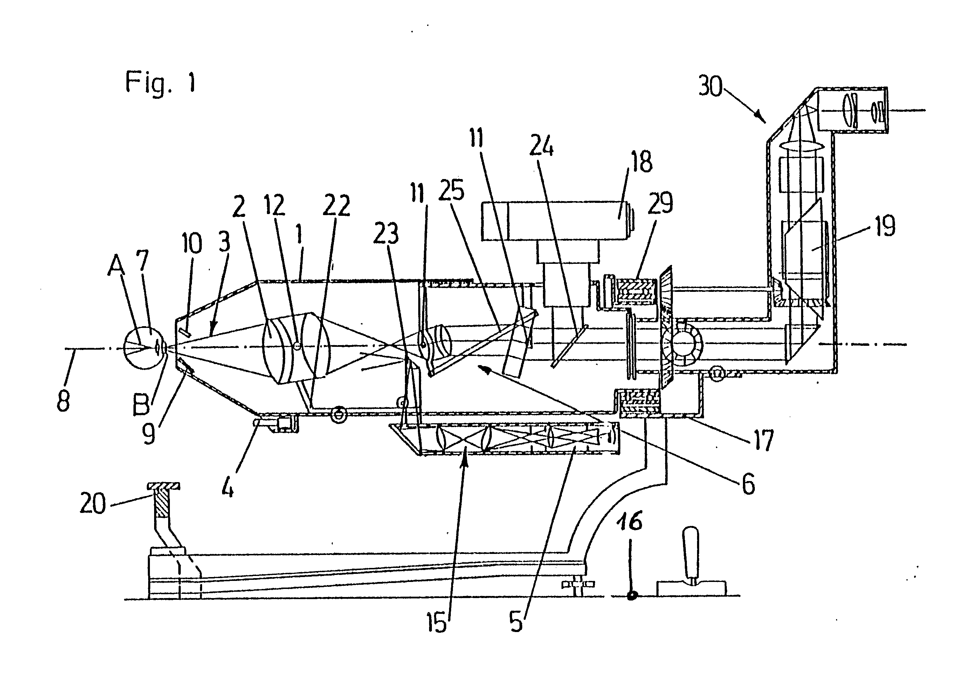

Am angrenzenden Ende des Gehäuses 1 ist eine Umlenkeinrichtung 3 vorgesehen, die in der Ausführung nach den Fig. 1-7 zwei Umlenkflächen 9, 10 aufweist.

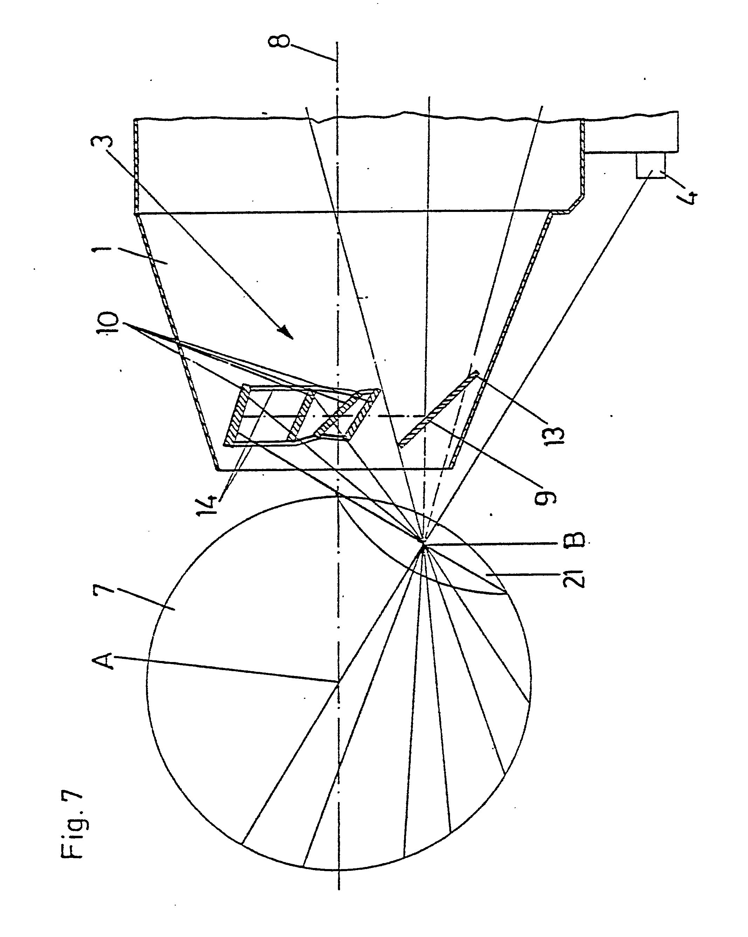

Die beiden Umlenkflächen 9, 10, insbesondere Umlenkspiegel der Umlenkeinrichtung 3, sind in Ihrer Lage verstellbar, um den Einfallswinkel auf die Augenlinse zu verändern. Die dem Objektiv nähere Umlenkfläche 9 ist zur Verstellung um eine Achse 13 kippbar angeordnet, während die andere, augennahe Umlenkfläche 10 auf Führungsbahnen 14 (Fig. 7) verschiebbar ist. Da die vordere Brennweite des Objektives in jeder Stellung der Umlenkflächen 9, 10 im wesentlichen unverändert sein soll, kann das Objektiv 2, wie erwähnt, verschoben werden.

Zur Fixierung der Augenachse ist eine Fixierobjekt 4, insbesondere ein Fixierlicht, vorgesehen, das exzentrisch zur Geräteachse 8 am Gehäuse mit diesem verdrehbar angeordnet ist. In der Untersuchungsstellung erscheint das Fixierobjekt 4 dem zu untersuchenden Auge 7, dessen Mittelpunkt A auf der Geräteachse 8 liegt, unter einem Winkel von etwa 35-40°, vorzugsweise 40°. Das Fixierobjekt 4 definiert mit der Geräteachse 8 eine Ebene, zu der die Drehachse 13 der Umlenkfläche 9, die Drehachse 12 des Objektives 2, sowie die Drehachse 11 des Zwischenlinsensystems 6 senkrecht stehen, und in der die Umlenkfläche 10 verschoben wird.

Die Untersuchung des Hintergrundes eines Auges 7 mit Hilfe des erfindungsgemäßen Gerätes erfolgt beispielsweise so : In der ersten Untersuchungsstellung (Fig. 1), in der das Kinn des Patienten auf der Kinnauflage 20 aufliegt, liegt, wie bereits erwähnt, der Augenmittelpunkt A in der Geräteachse 8 und die Augenachse fällt mit dieser zusammen. Die objektivnahe Umlenkfläche 9 ist hinuntergeklappt und die augennahe Umlenkfläche 10 hochgeschoben, und das Objektiv 2 entsprechend eingestellt, sodaß das Beobachtungsstrahlenbündel koaxial mit der Geräteachse 8 verläuft. Der dadurch erfaßbare zentrale Bereich des Augenhintergrundes umfaßt einen Beobachtungswinkel von ca. 17° von der Augenachse.

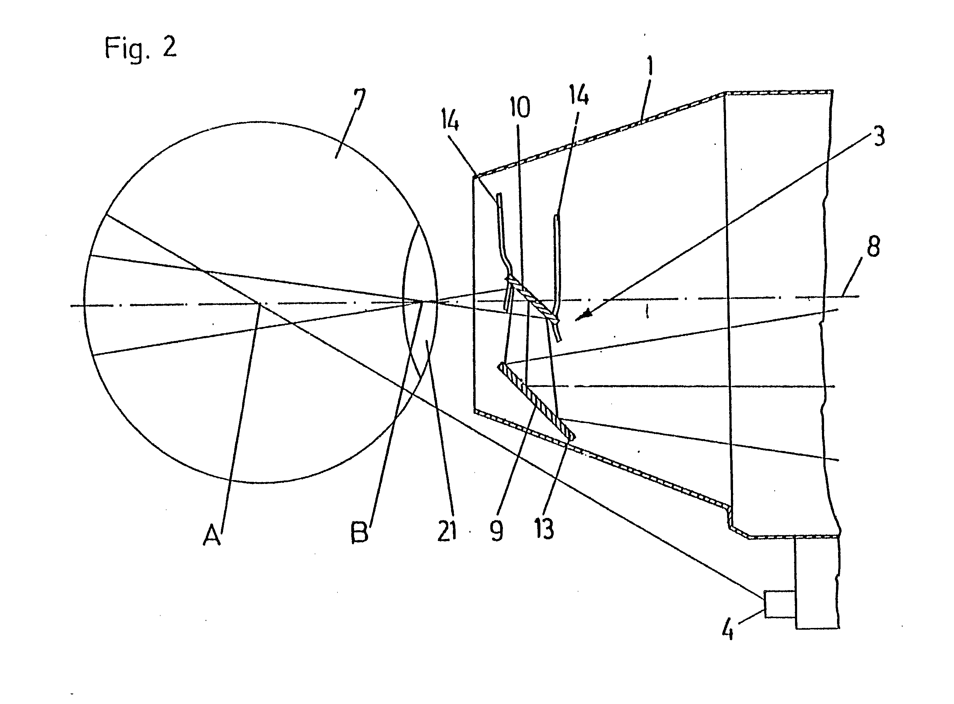

Das Objektiv 2 wird mittels der nicht näher dargestellten Verschiebeeinrichtung 22 vom vorderen Ende des Gehäuses 1 in entferntere Position verschoben, bis ein scharfes Bild vom Augenhintergrund erzielt wird. Eine zweite Ausgangsstellung zeigt vergrößert Fig. 2. Hier ist das nicht gezeigte Objektiv 2 etwas stärker geneigt, die objektivnahe Umlenkfläche 9 ist schräg hochgeklappt und die augennahe Umlenkfläche 10 in einer mittleren Stellung. Auch in dieser Ausführung verläuft das Beobachtungsstrahlenbündel von der augennahen Umlenkfläche 10 weg koaxial mit der Geräteachse 8.

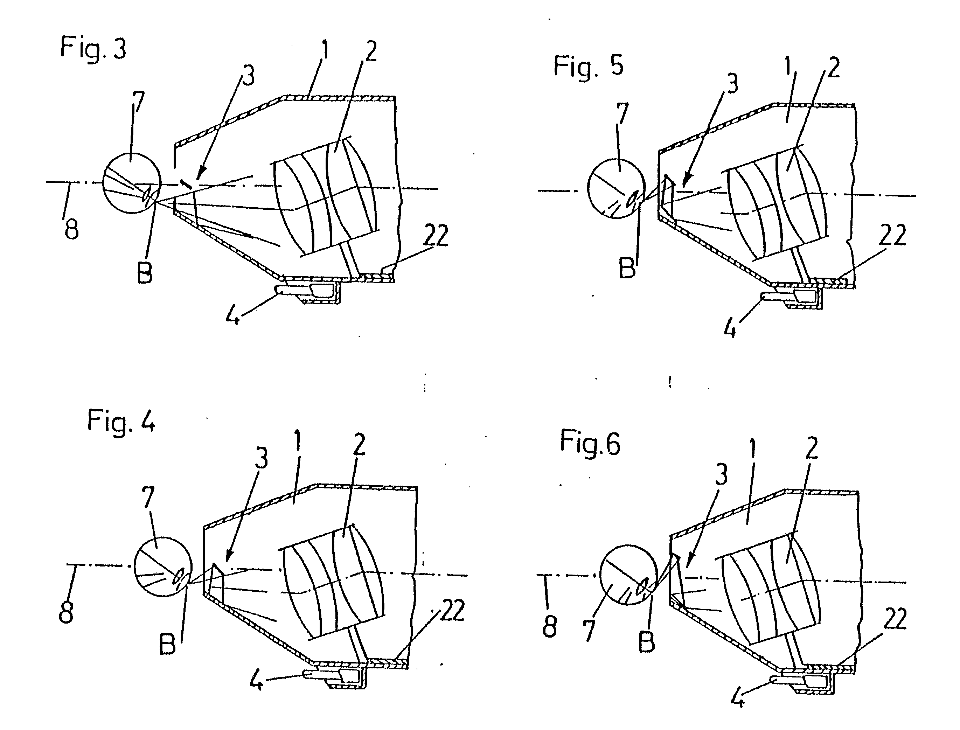

Für die Beobachtung des anschließenden Ringes des Augenhintergrundes von etwa 17°-45° von der Augenachse wird das Auge 7 auf das Fixierobjekt 4 gerichtet, sodaß die Augenachse um 35°-40°, vorzugsweise 40°, schräg genneigt ist. Diese zweite Untersuchungsstellung wird für die weitere Untersuchung beibehalten (Fig. 3-6) ; darin liegen der Abbildungspunkt B der Lichtquelle und das Fixierobjekt 4 in der Augenachse.

Für den Betrachtungswinkel zwischen 17° und 45° von der Augenachse wird nun die objektivnahe Umlenkfläche 9 nach unten geklappt, und die augennahe Umlenkfläche 10 in eine beliebige Position gebracht bzw. in der Stellung unverändert gelassen (Fig. 3). Das Beobachtungs- und Beleuchtungsstrahlenbündel erfährt auch hier, ähnlich Fig. 1, zwischen dem Abbildungspunkt B der Lichtquelle und dem Objektiv 2 keine Umlenkung und wird entsprechend dem Brechungsgesetz beim Eintritt in das Auge gebrochen, sodaß ein Auschnitt eines ersten Beobachtungsringes im nichtzentralen Bereich des Augenhintergrundes sichtbar ist.

Durch Drehen des Gehäuses 1 um die Geräteachse 8 werden das optische System mit der Umlenkeinrichtung 3, dem Objektiv 2 und dem Zwischenlinsensystem 6 sowie das Fixierobjekt 4 gedreht. Das auf das Fixierobjekt 4 gerichtete Auge 7 schwenkt dabei um den Augenmittelpunkt A mit, sodaß nacheinander alle Ausschnitte des ersten Betrachtungsringes sichtbar werden. Da die Ausschnitte des Augenhintergrundes Außerhalb des zentralen Bereiches nicht senkrecht zum Strahlenbündel, sondern geneigt dazu sind, ist zur Entzerrung des nicht tiefenscharfen Bildes das Zwischenbildlinsensystem 6 vorgesehen, das um die Achsen 11 kippbar ist und aus einer dem Objektiv 2 ähnlichen Linsenanordnung und einer damit bewegungsschlüssig über die Koppel 25 verbundenen planparallelen Platte besteht. Die Schrägstellungen des Objektives 2 und des Zwischenbildsystems 6 sind dabei so gewählt, daß für jeden Betrachtungswinkel die Scheimpflugbedingung erfüllt ist (Gegenstandsebene, Objektivebene und Abbildungsebene schneiden sich in einer Geraden).

Dadurch wird ein im wesentlichen entzerrtes Zwischenbild erhalten. Das Beleuchtungsstrahlenbündel durchläuft vor der Umlenkung zum Objektiv bevorzugt ebenfalls ein Zwischenlinsensystem 15, um auch hier die Scheimpflugbedindung zu erfüllen und durch scharfe Abbildung in der Pupille des zu untersuchenden Auges eine möglichst optimale Ausleuchtung des Ausschnittes am Augenhintergrund zu erzielen. Die Bewegung der Zwischenlinsensysteme 6 und 15 ist bevorzugt ebenfalls mit der Bewegung der Umlenkeinrichtung und des Objektives 2 gekoppelt, sodaß nicht jede Betrachtungsstellung (Fig. 4, 5, 6) einzeln justiert werden muß.

In Fig. 4 ist die Stellung der Umlenkflächen 9, 10 für den zweiten Betrachtungsring, in Fig. 5 für einen dritten und in Fig. 6 für den äußersten Betrachtungsring, wobei zur Betrachtung jedes Ringes eine vollständige Drehung des Gehäuses um die Geräteachse 8 erforderlich ist.

In Fig. 7 sind die beschriebenen Stellungen der Umlenkfläche 10 ineinander gezeichnet. Die einzelnen Stellungen können durch bloßes Verschieben der Umlenkfläche 10 in der durch die Geräteachse 8 und das Fixierobjekt 4 gegebenen Ebene erzielt werden, wobei die Umlenkfläche 10 vor allem an einem Spiegel ausgebildet ist, der in zwei nicht parallelen Führungsbahnen 14 verschiebbar ist.

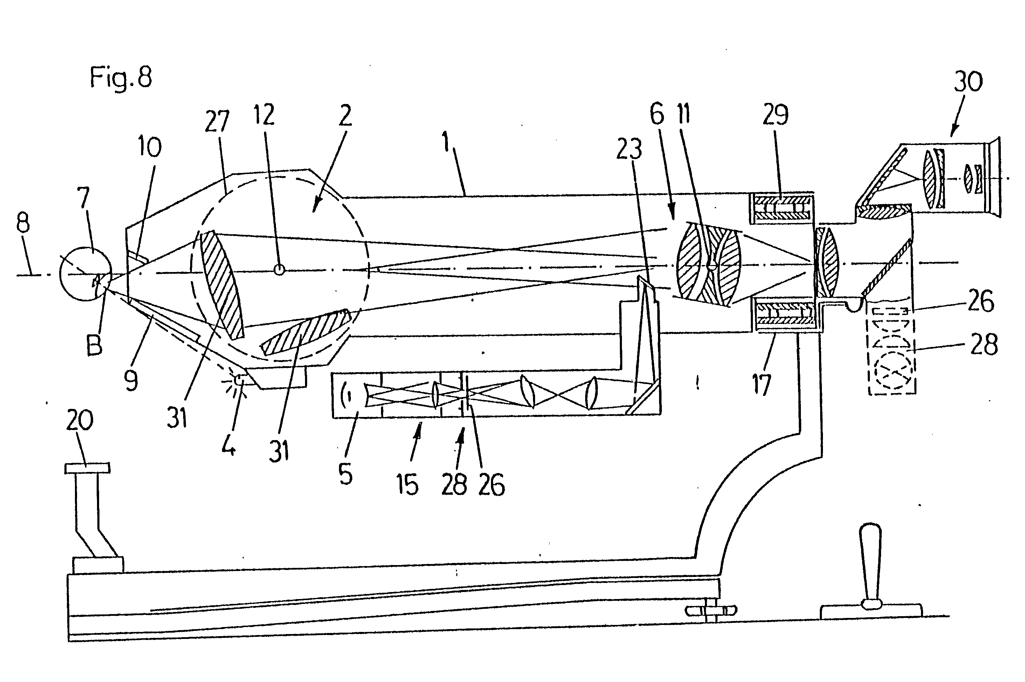

Fig. 8 zeigt eine weitere Ausführung, bei der das Objektiv 2 aus zumindest zwei unterschiedlichen Linsen 31 besteht, die in einem Käfig 27 um die Achse 12 drehbar angeordnet sind. Dadurch ist es möglich, einerseits die den verschiedenen Stellungen der Umlenkflächen entsprechenden Veränderungen der Strahlenbündel durch Wechsel der Objektlinsen zu erzielen, und weiters auch ein vereinfachtes Zwischenlinsensystem 6 einzusetzen, da jede der um die Achse 31 drehbaren Objektlinsen zur Erfüllung der Scheimpflugbedingung zur Entzerrung des Zwischenbildes mit herangezogen werden kann.

In Fig. 8 ist weiters auch die Möglichkeit gezeigt, daß in das Zwischenlinsensystem des Beleuchtungsstrahlenbündels und/oder in das Beobachtungsstrahlenbündel eine Diaeinrichtung 28 eingesetzt ist, sodaß Vergleichsbilder 26, beispielsweise von früheren Untersuchungen derselben Patienten einblendbar bzw. einspiegelbar sind.

Das erfindungsgemäße Gerät stellt einen indirekten Augenspiegel in Form eines Standgerätes dar, mit dem vor allem der wesentliche periphere Bereich des Augenhintergrundes betrachtbar und gegebenenfalls fotografierbar ist, ohne daß für den Patienten größere Unannehmlichkeiten, wie auf die Hornhaut aufzusetzende Linsen, Pupillenerweiterung usw. bestehen. Es ermöglicht weiters eine im wesentlichen entzerrte Darstellung auch der zum Strahlenbündel geneigten Ausschnitte des Augenhintergrundes sowie eine verstärkte reflexfreie Ausleuchtung des jeweiligen Ausschnittes. Ein zweites, mit dem ersten parallel geführtes Fixierobjekt für das zweite Auge kann gegebenfalls ebenso vorgesehen sein. Die Umlenkflächen 9, 10, insbesondere Umlenkspiegel können selbstverständlich nicht nur plan, sondern auch konvex oder konkav gewölbt sein.

Anstelle der entlang der Führungsbahnen 14 verschiebbaren Umlenkfläche 10 kann auch für jede bevorzugte Stellung eine eigene Umlenkfläche 10 vorgesehen sein, die unabhängig voneinander um Achsen kippbar sind, die zu den Achsen 11, 12 und 13 parallel liegen. Es kann dann die für die jeweilige Stellung benötigte Umlenkfläche in den Strahlengang eingeschwenkt werden.

**Patentansprüche**

1. Ophthalmoskopisches Gerät zur abschnittsweisen Untersuchung und Beobachtung des Augenhintergrundes, insbesondere des nichtzentralen Bereiches, mit einem ein Objektiv (2) aufweisenden und ein Beobachtungsstrahlenbündel erzeugenden, optischen System, mit einer Beleuchtungseinrichtung (5), deren Strahlenbündel das Objektiv im wesentlichen koaxial zum Beobachtungsstrahlenbündel verläßt und mit einem exzentrisch zur Achse des Gerätes angeordneten Fixierobjekt (4) zur Ausrichtung der Augenachse, wobei durch die Geräteachse (8) und das Fixierobjekt eine Ebene definiert ist, dadurch gekennzeichnet, daß das Gerät als Standgerät mit einem verstellbaren Objektiv (2) ausgebildet ist, wobei dem Objektiv (2) zumindest eine verstellbare Einrichtung (3) zur Umlenkung der beiden Strahlenbündel zugeordnet ist, mittels der das Bild (B) der Lichtquelle und das Bild der Pupille des Untersuchers unter unterschiedlichen Betrachtungswinkeln in die Pupille des zu untersuchenden Auges legbar ist, wenn der Mittelpunkt des auf das Fixierobjekt (4) gerichteten Auges (7) in der Geräteachse (8) liegt, und daß das optische System, die Beleuchtungseinrichtung (5), das Fixierobjekt (4) und die Umlenkeinrichtung (3) gemeinsam um die Geräteachse (8) drehbar sind.

2. Ophthalmoskopisches Gerät nach Anspruch 1, dadurch gekennzeichnet, daß die Umlenkeinrichtung (3) zumindest zwei verstellbare Umlenkflächen (9, 10) aufweist.

3. Ophthalmoskopisches Gerät nach.Anspruch 2, dadurch gekennzeichnet, daß eine Umlenkfläche (9) um eine zur genannten Ebene senkrechte Achse (13) kippbar ist.

4. Ophthalmoskopisches Gerät nach Anspruch 2 oder 3, dadurch gekennzeichnet, daß eine Umlenkfläche (10) auf zwei nicht parallelen Führungsbahnen (14) in der genannten Ebene verschiebbar ist.

5. Ophthalmoskopisches Gerät nach Anspruch 1, dadurch gekennzeichnet, daß das Objektiv (2) längs der Geräteachse (8) verschiebbar ist.

6. Ophthalmoskopisches Gerät nach Anspruch 1 oder 5, dadurch gekennzeichnet, daß das Objektiv (2) um eine zur genannten Ebene senkrechte Achse (12) kippbar ist.

7. Ophthalmoskopisches Gerät nach einem der Ansprüche 1, 5 oder 6, dadurch gekennzeichnet, daß dem Objektiv (2) zumindest ein kippbares Zwischenlinsensystem (6) zugeordnet ist.

8. Ophthalmoskopisches Gerät nach einem der Ansprüche 2 bis 7, dadurch gekennzeichnet, daß die Bewegungen der Umlenkflächen (9, 10), des Objektives (2) und des Zwischenlinsensystems (6) mechanisch gekoppelt sind.

9. Ophthalmoskopisches Gerät nach einem der Ansprüche 1-8, dadurch gekennzeichnet, daß in das Beleuchtungsstrahlenbündel oder das Beobachtungsstrahlenbündel zumindest ein Vergleichsbild (26) einspiegelbar ist.

**Claims**

1. Ophthalmoscope device for examining and observing the fundus of the eye in sections, in particular, the non-central area, comprising an optical system having an objective lens (2), for producing an observation beam, an illumination means (5), the beam of which leaves the objective lens substantially coaxially with respect to the observation beam, and a fixing object (4) which is arranged eccentrically with respect to the axis of the device, for aligning the axis of the eye, a plane being defined by the axis (8) of the device and the fixing object, characterized in that the device is in the form of a standing device having an adjustable objective lens (2), wherein associated with the objective lens (2) is at least one adjustable means (3) for deflecting the two beams, by means of which the image (B) of the light source and the image of the pupil of the person conducting the examination can be put into the pupil of the eye to be examined at different observation angles, when the centrepoint of the eye (7) which is directed to the fixing object (4) is located on the axis (8) of the device, and that the optical system, the illumination means (5), the fixing object (4) and the deflection means (3) are jointly rotatable about the axis (8) of the device.

2. Ophthalmoscope device according to claim 1, characterized in that the deflection means (3) has at least two adjustable deflection surfaces (9, 10).

3. Ophthalmoscope device according to claim 2, characterized in that a deflection surface (9) is tiltable about an axis (13) that is normal to said plane.

4. Ophthalmoscope device according to claim 2 or 3, characterized in that a deflection surface (10) is displaceable on two non-parallel guide tracks (14) in said plane.

5. Ophthalmoscope device according to claim 1, characterized in that the objective lens (2) is displaceable along the axis (8) of the device.

6. Ophthalmoscope device according to claim 1 or 5, characterized in that the objective lens (2) is tiltable about an axis (12) that is normal to said plane.

7. Ophthalmoscope device according to one of claims 1, 5 or 6, characterized in that at least one tiltable intermediate lens system (6) is associated with the objective lens (2).

8. Ophthalmoscope device according to one of claims 2 to 7, characterized in that the movements of the deflection surfaces (9, 10) of the objective lens (2) and the intermediate lens system (6) are mechanically coupled.

9. Ophthalmoscope device according to one of claims 1 to 8, characterized in that at least one comparison image (26) can be reflected into the illumination beam or the observation beam.

**Revendications**

1. Appareil ophthalmoscopique destiné à l'étude et à l'observation par secteurs du fond de l'œil, spécialement de la zone qui n'est pas centrale, avec un système optique présentant un objectif (2) et produisant un faisceau de rayons d'observation, ce système optique comportant un dispositif d'éclairage (5), dont le faisceau de rayons quitte l'objectif essentiellement coaxialement par rapport au faisceau de rayons d'observation, l'appareil comportant un objet de fixation (4), excentrique par rapport à l'axe de l'appareil et destiné à l'alignement de l'axe de l'œil, un plan étant défini par l'axe de l'appareil (8) et l'objet de fixation, caractérisé en ce que l'appareil est un appareil à pied à objectif (2) réglable, cet objectif comportant au moins un dispositif réglable (3) pour la déviation des deux faisceaux de rayons, ce dispositif permettant de mettre l'image (B) de la source lumineuse et l'image de la pupille de l'examinateur, sous différents angles d'observation, dans la pupille de l'œil à examiner, quand le centre de l'œil (7) dirigé sur l'objet de fixation (4) est dans l'axe de l'appareil (8) ; caractérisé enfin en ce que le système optique, le dispositif d'éclairage (5), l'objet de fixation (4) et le dispositif de déviation (3) sont logés ensemble tournant autour de l'axe de l'appareil (8).

2. Appareil ophthalmoscopique suivant la revendication 1, caractérisé en ce que le dispositif de déviation (3) comporte au moins deux surfaces de déviation réglables (9, 10).

3. Appareil ophthalmoscopique suivant la revendication 2, caractérisé en ce qu'une surface de déviation (9) peut basculer autour d'un axe (13) perpendiculaire au plan indiqué.

4. Appareil ophthalmoscopique suivant la revendication 2 ou 3, caractérisé en ce qu'une surface de déviation (10) peut être déplacée dans le plan indiqué sur deux glissières non parallèles (14).

5. Appareil ophtalmoscopique suivant la revendication 1, caractérisé en ce que l'objectif (2) est déplaçable le long de l'axe de l'appareil (8).

6. Appareil ophtalmoscopique suivant la revendication 1 ou 5, caractérisé en ce que l'objectif (2) est basculable autour d'un axe (12) perpendiculaire au plan indiqué.

7. Appareil ophtalmoscopique suivant une des revendications 1, 5 ou 6, caractérisé en ce que l'objectif (2) comporte au moins un système basculable de lentilles intermédiaires (6).

8. Appareil ophtalmoscopique suivant une des revendications 2 à 7, caractérisé en ce que les déplacements des surfaces de déviation (9, 10) de l'objectif (2) et du système intermédiaire de lentilles (6) sont couplés mécaniquement.

9. Appareil ophtalmoscopique suivant une des revendications 1 à 8, caractérisé en ce que dans le faisceau de rayons d'éclairage ou dans le faisceau de rayons d'observation on peut introduire par un jeu de miroir au moins une image comparative.

Fig. 1

Fig. 2

0 100 340

Fig. 5

Fig. 3

Fig. 6

Fig. 4

Fig. 7

0 100 340

Fig.8